# DEMANDE DE BREVET EUROPEEN

(11) **EP 1 810 661 A1**
(43) Date de publication de la demande: **25.07.2007**
(21) Numéro de dépôt: 07290049.1
(22) Date de dépôt: 15.01.2007
(51) Int. Cl.: A61K 8/86, A61K 8/39, A61Q 5/06, A61K 8/81, A61K 8/84, A61K 8/88, A61K 8/87

(54) **Composition cosmétique comprenant un polymère fixant non-ionique et un ester de polyéthyleneglycol et d'acide gras particulier, procédé de fixation de la coiffure et utilisations**

(30) Priorité: 20.01.2006 FR 0600557
(71) Demandeur: L'ORÉAL, 75008 Paris (FR)
(72) Inventeur: Vrignand, Sabine, 93210 LA Plaine (FR); Bebot, Cécile, 92110 Clichy (FR)
(74) Mandataire: Casalonga, Axel

(57) **Abrégé**

La présente demande concerne une composition cosmétique comprenant, dans un milieu cosmétiquement acceptable, au moins un polymère fixant non-ionique et au moins un ester de polyéthylèneglycol et d'acide gras de formule :
R₁CO-(OCH₂CH₂)ₙ₀-[OCH₂-CH(OR₂)-CH₂]ₙ₁-(OCH₂CH₂)ₙ₂-R₃ où
R₂ correspond à l'hydrogène ou à un groupe (CH₂CH₂O)ₙ₃COR₄
n1 est un entier égal à 0 ou 1 ;
n2 représente un entier allant de 2 à 300 ;
n3 représente un entier allant de 1 à 300 ;
n0 est un entier allant de 0 à 300
R₃ correspond à l'hydrogène, à un groupe hydroxyle ou à un groupe R₅COO,

R₁, R₄, R₅, indépendamment l'un de l'autre, correspondent à un groupe alkyle en C₁₀ à C₃₀ ou alkylène en C₁₀ à C₃₀.

La présente demande vise encore un procédé pour la mise en forme ou le maintien de la coiffure dans lequel cette composition cosmétique est mise en oeuvre ainsi que les utilisations de cette composition notamment pour la fixation et la mise en forme de la coiffure de manière durable.

## Description

La présente demande se rapporte à une composition cosmétique coiffante et à un procédé de fixation des fibres kératiniques, de préférence les fibres kératiniques humaines et en particulier les cheveux.

Les compositions cosmétiques pour la mise en forme et/ou le maintien de la coiffure les plus répandues sur le marché de la cosmétique sont des compositions essentiellement constituées d'une solution le plus souvent alcoolique ou hydroalcoolique et d'un ou plusieurs composants, appelés composants fixants, qui sont généralement des résines polymères dont la fonction est de former des soudures entre les cheveux. Ces composants fixants sont souvent formulés en mélange avec divers adjuvants cosmétiques.

Les compositions peuvent aussi se présenter sous forme de gels.

Les compositions cosmétiques peuvent être conditionnées soit dans un pot, dans un tube, soit dans un flacon pompe, soit dans un récipient aérosol approprié mis sous pression à l'aide d'un agent propulseur. Le système aérosol contient alors d'une part une phase liquide (ou jus) et d'autre part d'un agent propulseur.

Une fois appliquée sur les cheveux, la composition, contenant les composés fixants et un solvant approprié, sèche permettant la formation de soudures nécessaires à la fixation de la chevelure par les composants fixants. Les soudures doivent être suffisamment rigides pour assurer le maintien des cheveux, cependant elles doivent être également suffisamment fragiles pour que l'utilisateur puisse, en peignant ou en brossant la chevelure, les détruire sans heurter le cuir chevelu ni endommager les cheveux.

Les résines filmogènes classiques généralement utilisées en tant qu'agent fixant en milieu alcoolique présentent l'inconvénient de conférer de médiocres propriétés cosmétiques à la composition coiffante, en particulier le toucher obtenu par la mise en oeuvre des compositions à base de résines filmogènes n'est pas très satisfaisant.

De manière générale, les polymères fixants permettent de bien fixer la coiffure dans la forme désirée. Cependant, au cours de la journée, la coiffure est soumise à différentes déformations (passage de la main dans les cheveux, port d'une capuche...) qui peu à peu friabilisent le film de polymère et diminuent la tenue de la coiffure.

De manière surprenante et avantageuse, la demanderesse a découvert que l'utilisation d'une combinaison d'au moins un polymère fixant non-ionique et d'au moins un ester de polyéthylèneglycol et d'acide gras particulier permet de fixer la coiffure de façon satisfaisante et aussi de lui conférer une forme qui dure plus longtemps que les coiffures mises en forme au moyen d'une composition fixante classique.

Ces compositions permettent aussi de conférer aux cheveux des propriétés cosmétiques satisfaisantes.

D'autres caractéristiques, aspects, objets et avantages de la présente invention apparaîtront encore plus clairement à la lecture de la description et des exemples qui suivent.

La présente invention a pour objet une composition cosmétique comprenant, dans un milieu cosmétiquement acceptable, au moins un polymère fixant non-ionique différent des copolymères de type poly(vinylpyrrolidone/vinylacétate) et au moins un ester particulier de polyéthylèneglycol et d'acide gras.

De préférence la composition cosmétique est une composition capillaire, de manière préférée une composition cosmétique capillaire coiffante.

La composition cosmétique selon l'invention peut se présenter sous la forme d'un spray, d'une mousse ou d'un gel.

Un autre objet de la présente invention consiste en un procédé pour la mise en forme ou le maintien de la coiffure dans lequel la composition cosmétique selon l'invention est mise en oeuvre.

Un troisième objet de l'invention concerne les utilisations de cette composition cosmétique en tant que composition coiffante pour la fixation et le maintien des cheveux notamment pour conférer à la coiffure une bonne tenue dans le temps.

De préférence, les compositions selon la présente demande sont des compositions non lavantes (non détergentes), elles comprennent de préférence moins de, 4 % en poids de tensioactifs détergents et plus particulièrement moins de 1 % en poids par rapport au poids total de la composition et encore mieux elles n'en contiennent pas.

On entend par "tensioactif détergent" tout tensioactif anionique ou non-ionique, différent des esters de polyéthylène glycol et d'acide gras de l'invention.Le milieu cosmétiquement acceptable utilisé dans les compositions selon la présente invention est un milieu hydroalcoolique ou alcoolique.

L'alcool utilisé dans les compositions selon la présente invention est un alcool monohydroxylé et/ou un polyol. L'alcool monohydroxylé est choisi de préférence parmi les alcools inférieurs en C₁-C₄ comme l'éthanol, l'isopropanol, le tertiobutanol, le n-butanol.L'alcool particulièrement préféré est l'éthanol.

Parmi les polyols utilisables dans les compositions selon la présente invention, on peut citer, par exemple, le propylèneglycol, les polyéthylèneglycols, les éthers de polyols et leurs mélanges.La concentration en alcool dans les compositions selon la présente invention va de 0,1 à 99 %, de préférence de 0,5 à 50 % et de manière encore plus préférée de 1 à 30 % en poids par rapport au poids total de la composition.

De manière particulièrement préférée, la concentration en alcool va de 1 à 25 % en poids par rapport au poids total de la composition.

Les esters de polyéthylèneglycol et d'acide gras utilisables dans les compositions selon la présente demande correspondent à la formule :

R₁CO-(OCH₂CH₂)ₙ₀-[OCH₂-CH(OR₂)-CH₂]ₙ₁-(OCH₂CH₂)ₙ₂-R₃

où
R₂ correspond à l'hydrogène ou à un groupe (CH₂CH₂O)ₙ₃COR₄
n1 est un entier égal à 0 ou 1 ;
n2 représente un entier allant de 2 à 300 ;
n3 représente un entier allant de 1 à 300 ;
n0 est un entier allant de 0 à 300,
R₃ correspond à l'hydrogène, à un groupe hydroxyle ou à un groupe R₅COO,
R₁, R₄, R₅, indépendamment l'un de l'autre, correspondent à un groupe alkyle en C₁₀ à C₃₀ ou alkylène en C₁₀ à C₃₀.

A titre d'exemple, on utilise le distéarate de polyéthylèneglycol (150 OE), ou le monostéarate de glycéryle oxyéthyléné (200 OE).

La concentration de l'ester de polyéthylèneglycol et d'acide gras va de 0,01 % à 20 % en poids, de préférence de 0,1 à 15 % et de manière encore plus particulière de 1 à 10 % en poids par rapport au poids total de la composition.

Tous les polymères fixants non-ioniques et leurs mélanges utilisés dans le domaine capillaire peuvent être utilisés dans les compositions selon la présente demande.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle autres que les copolymères de type poly(vinylpyrrolidone/vinylacétate) tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations P
   RIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame tels que les homopolymères de vinylpyrrolidone, et tels que le polyvinylcaprolactame commercialisé sous la dénomination Luviskol^{®} PLUS par la société BASF ; et
- les -copolymères de vinyllactame tels que le copolymère poly(vinylpyrrolidone/vinyllactame) vendu sous le nom commercial Luvitec^{®} VPC 55K65W par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés non-ioniques, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

On peut également utiliser comme polymères fixants, des polyuréthanes fonctionnalisés ou non, siliconés ou non, non-ioniques, ou leurs mélanges.

Généralement, le ou les polymères fixants non-ioniques représentent de 0,1 à 20 %, de préférence de 1 à 15 %, en poids du poids total de la composition. Encore plus préférentiellement cette teneur va de 7 à 15 % en poids du poids total de la composition.

De préférence, le rapport pondéral polymère fixant non-ionique/ester de polyéthylèneglycol et d'acide gras des compositions selon la présente demande est supérieur ou égal à 0,6. Encore plus préférentiellement ce rapport est va de 0,6 à 10.

Les compositions selon la présente demande peuvent également comprendre un ou plusieurs polymères fixants additionnels anioniques, cationiques, amphotères ou non-ioniques différents des polymères fixants non-ioniques particuliers de type poly(vinylpyrrolidone/vinylacétate) décrits ci-dessus.

La concentration en polymères fixants additionnels est généralement comprise entre 0,1 à 20 %, de préférence de 1 à 15 %, en poids du poids total de la composition.

De manière avantageuse, la composition selon la présente demande comprend aussi au moins un agent épaississant, ionique ou non ionique, encore appelé " agents d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné). Ces agents d'ajustement de la rhéologie sont de préférence polymériques. Ces agents polymériques peuvent notamment être choisis parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères ou copolymères réticulés d'acide acrylique ou d'acide acrylamidopropanesulfonique et les polymères épaississants associatifs tels que décrits ci-dessous. L'agent polymérique est notamment choisi parmi les homopolymères ou copolymères réticulés d'acide acrylique ou d'acide acrylamidopropanesulfonique, et de préférence parmi les homopolymères ou copolymères réticulés d'acide acrylamidopropanesulfonique.

La concentration en agents épaississants peut varier d'environ 0,01 à 10 %, de préférence 0,1 à 5 %, et encore plus préférentiellement de 0,3 à 3 % en poids par rapport au poids total de la composition selon l'invention.

Les polymères associatifs sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs peuvent être de type anionique, cationique, amphotère ou non ionique.

Leur concentration peut varier d'environ 0,01 à 10 %, de préférence 0,1 à 5 % en poids par rapport au poids total de la composition selon l'invention.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = C R' CH₂ O Bₙ R (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60 % en poids d'acide acrylique et/ou d'acide méthacrylique, de 5 à 60 % en poids de (méth)acrylates d'alkyles inférieurs, de 2 à 50 % en poids d'éther d'allyl à chaîne grasse de formule (XV), et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Stéareth 10), notamment ceux vendus par la société ALLIED COLLOIDS sous les dénominations SALCARE SC80® et SALCARE SC90® qui sont des émulsions aqueuses à 30 % d'un terpolymère réticulé d'acide méthacrylique, d'acrylate d'éthyle et de stéareth-10-allyl éther (40/50/ 10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (XVI) suivante : 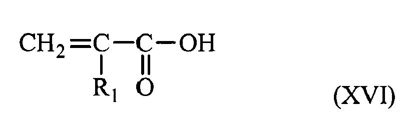 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante 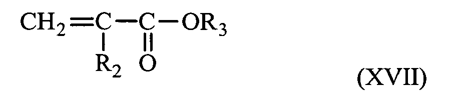 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US 3,915,921 et 4,509,949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (XVII) décrite ci-dessus et dans laquelle R₂ désigne H ou CH₃, R₃ désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères associatifs anioniques, on utilisera plus particulièrement ceux constitués de 95 à 60 % en poids d'acide acrylique (motif hydrophile), 4 à 40 % en poids d'acrylate d'alkyles en C₁₀-C₃₀ (motif hydrophobe), et 0 à 6 % en poids de monomère polymérisable réticulant, ou bien ceux constitués de 98 à 96 % en poids d'acide acrylique (motif hydrophile), 1 à 4 % en poids d'acrylate d'alkyles en C₁₀-C₃₀ (motif hydrophobe), et 0,1 à 0,6 % en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20 % à 70 % en poids d'un acide carboxylique à insaturation α,β-monoéthylénique,
   (b) environ 20 à 80 % en poids d'un monomère à insaturation α,β-monoéthylénique non-tensio-actif différent de (a),
   (c) environ 0,5 à 60 % en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhénylique éthoxylé (40 OE) en dispersion aqueuse à 25 % en poids.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (XVIII) suivante :

   R-X-(P)ₙ-[L-(Y)ₘ]ᵣ-L'-(P')ₚ-X'-R' (XVIII)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
   la molécule contenant au moins une fonction amine protonée ou quaternisée et au moins un groupement hydrophobe.
   Dans un mode de réalisation préféré de ces polyuréthanes, les seuls groupements hydrophobes sont les groupes R et R' aux extrémités de chaîne.
   Une famille préférée de polyuréthanes associatifs cationiques est celle correspondant à la formule (XVIII) décrite ci-dessus et dans laquelle :
   R et R' représentent tous les deux indépendamment un groupement hydrophobe,
   X, X' représentent chacun un groupe L",
   n et p valent entre 1 et 1000 et
   L, L', L", P, P', Y et m ont la signification indiquée ci-dessus.
   Une autre famille préférée de polyuréthanes associatifs cationiques est celle correspondant à la formule (XVIII) ci-dessus dans laquelle :
   R et R' représentent tous les deux indépendamment un groupement hydrophobe, X, X' représentent chacun un groupe L", n et p valent 0, et L, L', L", Y et m ont la signification indiquée ci-dessus.
   Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.
   Encore une autre famille préférée de polyuréthanes associatifs cationiques est celle correspondant à la formule (XVIII) ci-dessus dans laquelle :
   R et R' représentent tous les deux indépendamment un groupement hydrophobe,
   X et X' représentent tous les deux indépendamment un groupement comportant une amine quaternaire,
   n et p valent zéro, et
   L, L', Y et m ont la signification indiquée ci-dessus.
   La masse moléculaire moyenne en nombre des polyuréthanes associatifs cationiques est comprise de préférence entre 400 et 500 000, en particulier entre 1000 et 400 000 et idéalement entre 1000 et 300 000.
   Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
   Préférentiellement, le groupement hydrocarboné provient d'un composé monofonctionnel.
   A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
   Lorsque X et/ou X' désignent un groupement comportant une amine tertiaire ou quaternaire, X et/ou X' peuvent représenter l'une des formules suivantes : 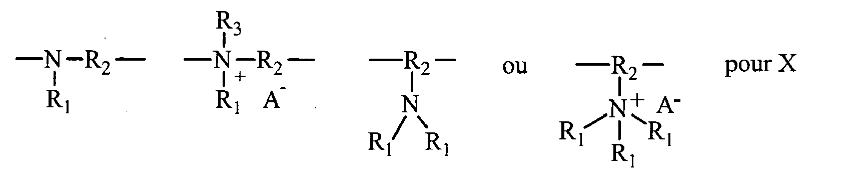 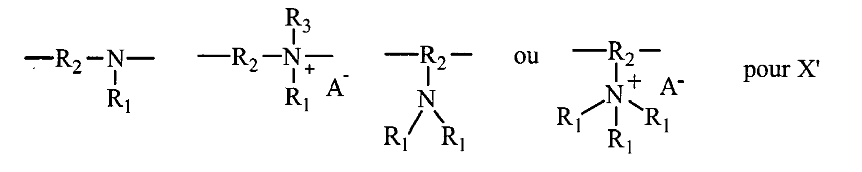 dans lesquelles :
   R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
   R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
   A- est un contre-ion physiologiquement acceptable.
   Les groupements L, L' et L" représentent un groupe de formule : 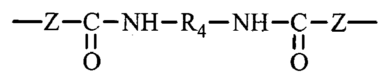 dans laquelle :
   Z représente -O-, -S- ou -NH- ; et
   R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.
   Les groupements P et P', comprenant une fonction amine peuvent représenter au moins l'une des formules suivantes : 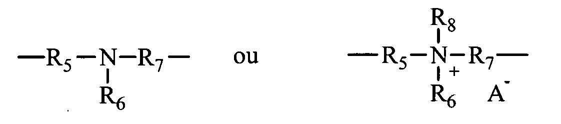 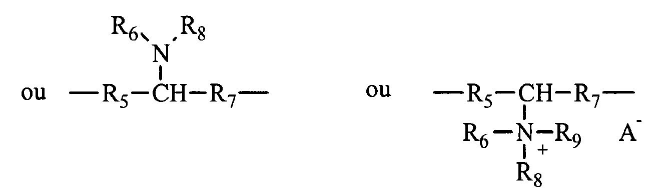 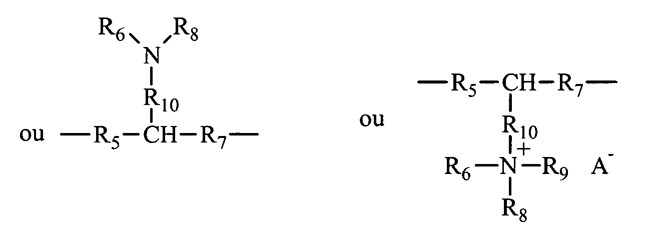 dans lesquelles :
   R₅ et R₇ ont les mêmes significations que R₂ défini précédemment;
   R₆, R₈ et R₉ ont les mêmes significations que R₁ et R₃ définis précédemment ;
   R₁₀ représente un groupe alkylène, linéaire ou ramifié, éventuellement insaturé et pouvant contenir un ou plusieurs hétéroatomes choisis parmi N, O, S et P,
   et A⁻ est un contre-ion physiologiquement acceptable.
   En ce qui concerne la signification de Y, on entend par groupement hydrophile, un groupement hydrosoluble polymérique ou non.
   A titre d'exemple, on peut citer, lorsqu'il ne s'agit pas de polymères, l'éthylèneglycol, le diéthylèneglycol et le propylèneglycol.
   Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Les polyuréthanes associatifs cationiques de formule (XVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.
   Un premier type de composés entrant dans la préparation du polyuréthane de formule (XVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
   Comme indiqué précédemment, ce composé peut comporter plus d'un motif à fonction amine. Il s'agit alors d'un polymère portant une répétition du motif à fonction amine.
   Ce type de composés peut être représenté par l'une des formules suivantes :

   HZ-(P)ₙ-ZH,

   ou

   HZ-(P')p-ZH

   dans lesquelles Z, P, P', n et p sont tels que définis plus haut.
   A titre d'exemple de composé à fonction amine, on peut citer la N-méthyldiéthanolamine, la N-tert-butyl-diéthanolamine, la N-sulfoéthyldiéthanolamine.
   Le deuxième composé entrant dans la préparation du polyuréthane de formule (XVIII) est un diisocyanate correspondant à la formule :

   O=C=N-R₄-N=C=O

   dans laquelle R₄ est défini plus haut.
   A titre d'exemple, on peut citer le méthylènediphényl-diisocyanate, le méthylènedicyclohexanediisocyanate, l'isophorone-diisocyanate, le toluènediisocyanate, le naphtalènediisocyanate, le butanediisocyanate, l'hexanediisocyanate.
   Un troisième composé entrant dans la préparation du polyuréthane de formule (XVIII) est un composé hydrophobe destiné à former les groupes hydrophobes terminaux du polymère de formule (XVIII).
   Ce composé est constitué d'un groupe hydrophobe et d'une fonction à hydrogène labile, par exemple une fonction hydroxyle, amine primaire ou secondaire, ou thiol.
   A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.
   Le groupe hydrophobe du polyuréthane de formule (XVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.
   Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.
   Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
   A titre d'exemple, on peut citer, lorsqu'il ne s'agit pas de polymères, l'éthylèneglycol, le diéthylèneglycol et le propylèneglycol.
   Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Le groupe hydrophile noté Y dans la formule (XVIII) est facultatif. En effet, les motifs à fonction amine quaternaire ou protonée peuvent suffire à apporter la solubilité ou l'hydrodispersibilité nécessaire pour ce type de polymère dans une solution aqueuse.
   Bien que la présence d'un groupe Y hydrophile soit facultative, on préfère cependant des polyuréthanes associatifs cationiques comportant un tel groupe.
-(II) les dérivés de cellulose quaternisée et les polyacrylates à groupements latéraux aminés non cycliques.

Les dérivés de cellulose quaternisée sont en particulier :
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

Les polymères associatifs amphotères sont choisis de préférence parmi ceux comportant au moins un motif cationique non cyclique. Plus particulièrement encore, on préfère ceux préparés à partir ou comprenant 1 à 20 % en moles de monomère comportant une chaîne grasse, et de préférence 1,5 à 15 % en moles et plus particulièrement encore 1,5 à 6 % en moles, par rapport au nombre total de moles de monomères.

Les polymères associatifs amphotères préférés selon l'invention comprennent, ou sont préparés en copolymérisant :
1) au moins un monomère de formule (XIX) ou (XX) : 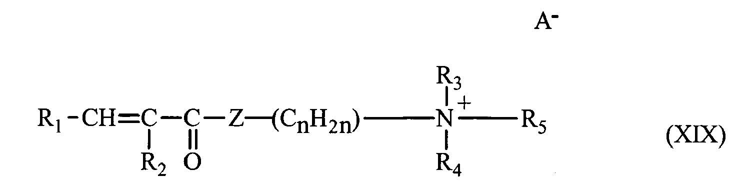 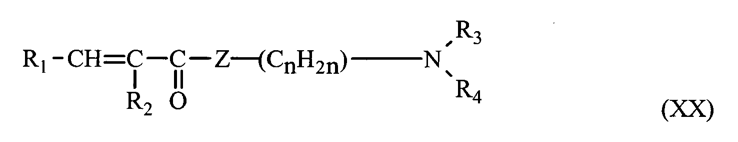 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (XXI) :

   R₆-CH =CR₇-COOH (XXI)

   dans laquelle R₆ et R₇, identiques ou différents, représentent un atome d'hydrogène ou un radical méthyle; et
3) au moins un monomère de formule (XXII) :

   R₆-CH =CR₇-COXR₈ (XXII)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (XIX), (XX) ou (XXII) comportant au moins une chaîne grasse.

Les monomères de formule (XIX) et (XX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (XIX) est choisi parmi le chlorure d'acrylamidopropyl triméthyl ammonium et le chlorure de méthacrylamidopropyl triméthyl ammonium.

Les monomères de formule (XXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXI) est l'acide acrylique.

Les monomères de formule (XXII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % en moles du monomère comportant une chaîne grasse (monomère de formule (XIX), (XX) ou (XXII)), et de préférence de 1,5 à 6 % en moles.

Les poids moléculaires moyens en poids des polymères associatifs amphotères selon l'invention peuvent varier de 500 à 50 000 000 et sont de préférence compris entre 10 000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO 98/44012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
   - (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
   - (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
   - (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
   - (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380-389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15 % en poids dans une matrice de maltodextrine (4 %) et d'eau (81 %); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

De manière générale, la concentration en polymère épaississant va de 0,1 à 5 %, de préférence de 0,3 à 3 % en poids par rapport au poids total de la composition.

La composition peut contenir en outre au moins un additif choisi parmi les principes actifs et adjuvants cosmétiques utilisés couramment dans le domaine capillaire. Ces additifs sont choisis par exemple parmi les vitamines, les acides aminés, les oligopeptides, les peptides, les protéines hydrolysées ou non, modifiées ou non, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs ioniques ou non ioniques, les silicones, les huiles minérales, végétales ou animales, les polyisobutènes et poly(α-oléfines), les esters gras additionnels autres que les esters de polyéthylèneglycol et d'acide gras mentionnés plus haut, les agents colorants capillaires tels que les colorants directs, précurseurs de colorant par oxydation et les pigment, les acides, bases, plastifiants, parfums, conservateurs, charges minérales, nacres, paillettes.

Ces additifs sont présents dans la composition selon l'invention en une quantité allant de 0 à 20 % en poids par rapport au poids total de la composition.

Bien entendu, l'homme de l'art veillera à choisir ce ou ces principes actifs et adjuvants cosmétiques complémentaires de manière telle que les propriétés avantageuses attachées intrinsèquement au dispositif et procédé conformes à l'invention ne soient pas, ou substantiellement pas, altérées par la ou les adjonctions envisagées.

Les exemples qui suivent servent à illustrer l'invention sans toutefois présenter un caractère limitatif.

### EXEMPLES

On a réalisée les compositions suivantes (en % de matière active) :

| Nom INCI | Ex 1 | Ex 2 |
|---|---|---|
| PVP | 5 % | 6 % |
| PEG-100 Stéarate | | 2 % |
| PEG-150 Distéarate | 2 % | |
| PEG-200 Glycéryl Stéarate | 5 % | 4 % |
| Glycérol | | 2 % |
| Propylène Glycol | 1,5 % | |
| SEPIGEL305 (polymère d'acide acrylamidopropane sulfonique en émulsion inverse) | 0,3 % | 1 % |
| Carbomer | | 1 % |
| PEG-40 huile de castor hydrogénée | 0,5 % | 0,5 % |
| Ethanol | | 20 % |
| Conservateurs, Neutralisant, Parfum | QS | QS |
| Eau | QS100 | QS100 |

Ces compositions présentent de bonnes propriétés fixantes ainsi qu'une rémanence de ces propriétés dans le temps.

## Revendications

1. Composition cosmétique comprenant, dans un milieu cosmétiquement acceptable, au moins un polymère fixant non-ionique différent des copolymères de type poly(vinylpyrrolidone/vinylacétate) et au moins un ester de polyéthylèneglycol et d'acide gras de formule :
R₁CO-(OCH₂CH₂)ₙ₀-[OCH₂-CH(OR₂)-CH₂]ₙ₁-(OCH₂CH₂)ₙ₂-R₃
où
R₂ correspond à l'hydrogène ou à un groupe (CH₂CH₂O)ₙ₃COR₄
n1 est un entier égal à 0 ou 1 ;
n2 représente un entier allant de 2 à 300 ;
n3 représente un entier allant de 1 à 300 ;
n0 est un entier allant de 0 à 300
R₃ correspond à l'hydrogène, à un groupe hydroxyle ou à un groupe R₅COO,
R₁, R₄, R₅, indépendamment l'un de l'autre, correspondent à un groupe alkyle en C₁₀ à C₃₀ ou alkylène en C₁₀ à C₃₀.

2. Composition selon la revendication 1 telle que le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères et copolymères de vinyllactame, les polyuréthanes non ioniques, et les polymères siliconés greffés non ioniques.

3. Composition selon la revendication 2 telle que les homopolymères de vinyllactame sont des homopolymères de polyvinylpyrrolidone.

4. Composition cosmétique selon l'une des revendications précédentes telle que la concentration en polymère fixant non-ionique va de 0,1 à 20 %, de préférence de 1 à 15 % en poids du poids total de la composition.

5. Composition selon la revendication précédente telle que la concentration en polymère fixant non-ionique va de 7 à 15 % en poids du poids total de la composition.

6. Composition cosmétique selon l'une des revendications précédentes telle que la concentration en ester de polyéthylèneglycol et d'acide gras va de 0,01 % à 20 % en poids, de préférence de 0,1 à 15 % et de manière encore plus particulière de 1 à 10 % en poids par rapport au poids total de la composition.

7. Composition cosmétique selon l'une des revendications précédentes telle que telle que le rapport pondéral polymère fixant non-ionique/ester de polyéthylèneglycol et d'acide gras est supérieur ou égal à 0,6.

8. Composition cosmétique selon la revendication précédente telle que le rapport pondéral polymère fixant non-ionique/ester de polyéthylèneglycol et d'acide gras va de 0,6 à 10.

9. Composition cosmétique selon l'une des revendications précédentes telle qu'elle comprend au moins un agent épaississant.

10. Composition cosmétique selon la revendication précédente telle que l'agent épaississant est un polymère épaississant.

11. Composition cosmétique selon la revendication précédente **caractérisée en ce qu'**elle comprend un polymère épaississant associatif de type anionique contenant au moins un motif hydrophile de type acide carboxylique insaturé oléfinique, et au moins un motif hydrophobe de type ester d'alkyl (C₁₀-C₃₀) d'acide carboxylique insaturé.

12. Composition cosmétique selon la revendication précédente telle que la concentration en polymère épaississant va de 0,01 à 10 %, de préférence de 0,1 à 5 % et de manière encore préférée de 0,3 à 3 % en poids par rapport au poids total de la composition.

13. Composition cosmétique selon l'une des revendications précédentes telle qu'elle comprend au moins un additif choisi parmi les vitamines, les acides aminés, les oligopeptides, les peptides, les protéines hydrolysées ou non, modifiées ou non, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs ioniques ou non ioniques, les silicones, les huiles minérales, végétales ou animales, les polyisobutènes et poly(α-oléfines), les esters gras additionnels, les agents colorants capillaires tels que les colorants directs, précurseurs de colorant par oxydation et les pigment, les acides, bases, plastifiants, parfums, conservateurs, charges minérales, nacres, paillettes.

14. Composition cosmétique selon l'une des revendications précédentes telle qu'elle se présente sous la forme d'un spray.

15. Composition cosmétique selon l'une des revendications 1 à 13 telle qu'elle se présente sous la forme d'un gel.

16. Composition cosmétique selon l'une des revendications 1 à 13 telle qu'elle se présente sous la forme d'une mousse.

17. Procédé pour la mise en forme ou le maintien de la coiffure dans lequel la composition cosmétique selon l'une des revendications 1 à 16 est mise en oeuvre.

18. Utilisation de la composition cosmétique selon l'une des revendications 1 à 16 en tant que composition coiffante pour la fixation et le maintien des cheveux.

19. Utilisation de la composition cosmétique selon la revendication précédente pour conférer à la coiffure une bonne tenue dans le temps.
